# EUROPEAN PATENT APPLICATION

(11) **EP 4 361 258 A1**
(43) Date of publication of application: **01.05.2024**
(21) Application number: 22828024.4
(22) Date of filing: 29.03.2022
(51) Int. Cl.: C12N 7/02

(54) **VIRUS SAMPLE CONCENTRATING METHOD**

(30) Priority: 24.06.2021 JP 2021105046
(71) Applicant: Shimadzu Corporation, Nakagyo-ku, Kyoto-shi, Kyoto 604-8511 (JP); Kyoto University, Kyoto-shi, Kyoto 606-8501 (JP)
(72) Inventor: YASOJIMA, Makoto, Kyoto-shi, Kyoto 604-8436 (JP); TAKEMORI, Hiroaki, Kyoto-shi, Kyoto 604-8436 (JP); TOMONO, Takuya, Kyoto-shi, Kyoto 604-8436 (JP); DAIGO, Fumi, Kyoto-shi, Kyoto 604-8436 (JP); TANAKA, Hiroaki, Kyoto-shi, Kyoto 606-8501 (JP); IHARA, Masaru, Kyoto-shi, Kyoto 606-8501 (JP)
(74) Representative: Müller-Boré & Partner Patentanwälte PartG mbB
(86) International application number: PCT/JP2022/015580
(87) International publication number: WO 2022/270105

(57) **Abstract**

A liquid (30) containing a virus passes through a solid phase (10) made of a polymer having a hydrophobic group to cause the solid phase (10) to retain viruses (40), and then an organic solvent (70) causes the viruses (40) to be eluted from the solid phase (10). As a result, it is possible to rapidly concentrate a virus sample at a high concentration rate.

## Description

### TECHNICAL FIELD

The present invention relates to a method for concentrating a sample containing viruses.

### BACKGROUND ART

There is a field of study called sewage epidemiology which attempts to grasp the development trend of infectious diseases by measuring virus concentration in sewage including human urine and feces. A large number of results from sewage epidemiology have been reported in infection control measures against norovirus and poliovirus, and it is considered that the results can be applied to novel coronavirus infectious diseases (COVID-19) in which infection spread has become serious in recent years.

A virus in a sample is detected by a nucleic acid amplification method such as polymerase chain reaction (PCR) or reverse transcription PCR (RT-PCR) using primers specific to the virus. However, since the virus concentration in sewage is generally low and often falls below a detection limit of the nucleic acid amplification method, it is necessary to concentrate a sample containing viruses in advance.

Conventionally, as a concentration technique for a virus sample, a concentration precipitation method (what is called PEG precipitation method) by adding polyethylene glycol (PEG) is known. For example, Non Patent Literature 1 has description that the virus concentration in a sewage sample is increased by the PEG precipitation method.

In addition, in recent years, a method for concentrating a virus sample using a solid phase extraction method has also been proposed. The solid phase extraction method is a technique for selectively isolating or concentrating a target substance in a liquid by causing the target substance to interact with a solid phase. For example, Patent Literature 1 has description that it is possible to concentrate a virus sample by passing water containing viruses through a column packed with a solid phase having anion exchange ability to allow the carrier to adsorb the viruses, and then passing an alkaline solution (buffer solution with a pH of 9 or higher) into the column to cause the viruses to be eluted.

### CITATION LIST

### PATENT LITERATURE

Patent Literature 1: JP 2004-129548 A

### NON PATENT LITERATURE

Non Patent Literature 1: Lewis and Metcalf, 1988, "Polyethylene Glycol Precipitation for Recovery of Pathogenic Viruses, Including Hepatitis A Virus and Human Rotavirus, from Oyster, Water, and Sediment Samples", Appl. Environ. Microbiol. vol 54(8), p. 1983-1988

### SUMMARY OF INVENTION

### TECHNICAL PROBLEM

However, since the number of infected people is small in an early stage of infectious diseases or a convergence stage, the amount of virus discharged into sewage is inevitably small. Therefore, in order to sharply grasp an infection status in such a period, a technique capable of concentrating a virus sample at a higher concentration rate is required. In addition, in order to utilize sewage epidemiology for infection spread countermeasures, it is necessary to quickly obtain a result indicating the infection status, and thus a technique capable of concentrating a virus sample in a shorter time has been required.

The present invention has been made in view of the above points, and an object of the present invention is to provide a method for concentrating a virus sample capable of rapidly concentrating the virus sample at a high concentration rate.

### SOLUTION TO PROBLEM

A method according to the present invention which has been made to solve the above problem is a method for concentrating a virus sample, including
bringing a liquid containing a virus into contact with a solid phase made of a polymer having a hydrophobic group to allow the solid phase to retain the virus, and then bringing an organic solvent into contact with the solid phase retaining the virus to cause the virus to be eluted from the solid phase.

### ADVANTAGEOUS EFFECTS OF INVENTION

With the method for concentrating a virus sample according to the present invention, it is possible to rapidly concentrate a virus sample at a high concentration rate.

### BRIEF DESCRIPTION OF DRAWINGS

[Fig. 1] Fig. 1 is a schematic view illustrating a virus adsorption step on a solid phase in one embodiment of the present invention.
[Fig. 2] Fig. 2 is a schematic view illustrating a virus elution step in the same embodiment.
[Fig. 3] Fig. 3 is a schematic view illustrating a nitrogen blowing step in the same embodiment.
[Fig. 4] Fig. 4 includes graphs representing quantitative results of the N1 gene in an example of the present invention.

### DESCRIPTION OF EMBODIMENTS

The method for concentrating a virus sample according to the present embodiment (hereinafter referred to as "method of the present embodiment") is to increase the virus concentration in a liquid sample using a solid phase extraction method. In the method of the present embodiment, a solid phase made of a polymer having a hydrophobic group is used as a solid phase for adsorbing a virus. As such a solid phase, for example, a hydrophilic lipophilic balanced solid phase (HLB solid phase) can be suitably used, but the solid phase is not limited to the this. In addition, in the method of the present embodiment, an organic solvent is used as a solvent for eluting the virus adsorbed on the solid phase (hereinafter referred to as "solvent for elution"). As the organic solvent, for example, an alcohol solvent such as methanol or ethanol can be suitably used, but the organic solvent in the present invention is not limited to this. Note that examples of the virus targeted in the method of the present embodiment include, but are not limited to, a coronavirus such as a novel coronavirus (SARS-CoV-2) or an enveloped virus such as an influenza virus. For example, the method of the present embodiment can also be applied to concentration of a sample containing a non-enveloped virus such as a norovirus or a poliovirus. In addition, the "liquid containing a virus" in the method of the present embodiment is typically sewage water, but is not limited to this, and may be, for example, river water, sea water, a liquid containing body fluid collected from a human, or the like.

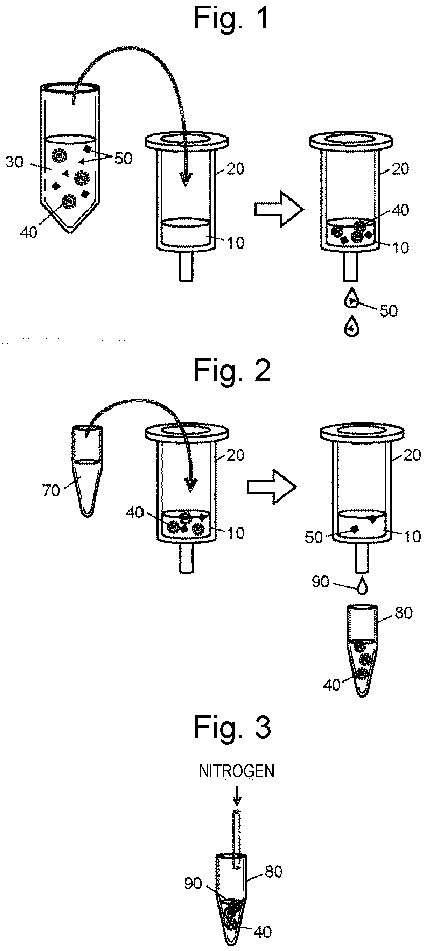

Hereinafter, a procedure of standard processing for virus concentration in the method of the present embodiment will be described with reference to Figs. 1 to 3.

### (1) Adsorption of Virus on Solid Phase

First, a conditioning liquid (for example, methanol and pure water) is caused to flow through a cartridge 20 filled with a solid phase 10 made of a polymer having a hydrophobic group to activate the solid phase 10. Note that examples of the method for causing a liquid to flow through the solid phase 10 include a pressurization method using a syringe or the like, a suction method using a suction manifold and a vacuum pump, a centrifugal separation method, and a natural drop method. In the method of the present embodiment, any desired method may be used, but it is desirable to adopt a suction method capable of passing water in a short time and having less possibility of sample contamination. Thereafter, a sewage sample 30 collected from a predetermined place is passed through the cartridge 20 (Fig. 1). As a result, viruses 40 in the sewage sample 30 are adsorbed on the solid phase 10 made of a polymer having a hydrophobic group. On the other hand, some of contaminants 50 in the sewage sample 30 flow out of the cartridge 20 without being adsorbed on the solid phase 10. The contaminants 50 are powders or fine particles, and such contaminants 50 may inhibit virus detection by PCR or the like described later. In the method of the present embodiment, in a process of adsorbing the viruses 40 on the solid phase 10 (and a process of eluting the viruses 40 from the solid phase 10 described later), the contaminants 50 can be separated from the viruses 40, so that final improvement in virus detection accuracy can also be expected. Note that it is desirable that contaminants having a relatively large specific gravity or size are removed in advance from the sewage sample 30 before flowing into the cartridge 20 by performing centrifugal separation or filtration.

### (2) Elution of Virus from Solid Phase

Subsequently, a solvent (for example, pure water) with which contaminants are eluted, but the virus 40 is not eluted is passed through the cartridge 20 to wash out the contaminants in the solid phase, and then, an organic solvent 70 flows through the cartridge 20 to cause the virus 40 adsorbed on the solid phase 10 to be eluted and recovered in a predetermined recovery container 80 (Fig. 2). An amount of the organic solvent 70 used in this step is set to be smaller (for example, an amount of about 1/100 to 1/20 of the sewage sample 30) than that of the sewage sample 30 flowing into the cartridge 20 in the step (1) described above. As a result, an eluate 90 eluted from the cartridge 20 contains the virus 40 at a higher concentration than the sewage sample 30. Note that the contaminants 50 not eluted by the organic solvent in this step remain in the cartridge 20 while being adsorbed on the solid phase 10. Note that it is known that when a virus is reacted with an organic solvent such as alcohol, the virus is destroyed. However, as a result of intensive studies, the present inventor has found that even when the viruses 40 adsorbed on the solid phase 10 are eluted by the organic solvent 70 as in the method of the present embodiment, it is possible to elute the viruses 40 without destroying viral nucleic acids (DNA or RNA) necessary for virus detection by PCR or the like, which will be described later. In addition, in a series of processing for eluting viruses from the solid phase 10, it can be expected that some of the viruses 40 being processed are detoxified by the organic solvent 70, and a risk of virus infection of a practitioner can be reduced.

### (3) Blowing Nitrogen

By blowing nitrogen gas onto the eluate 90 obtained as described above, part of the organic solvent in the eluate 90 is evaporated. As a result, the concentration of the viruses 40 in the eluate 90 can be further increased. Since most of the eluate 90 is an organic solvent having high volatility, a high volatilization effect can be obtained as compared with a case of performing extraction using water. Note that, in this step, nitrogen gas may be blown while the eluate 90 is being heated. In this case, the organic solvent can be evaporated in a shorter time. Note that the temperature in a case of heating the eluate 90 is desirably about 35 °C to 41 °C so that the nucleic acids (DNAs or RNAs) contained in the viruses 40 are not destroyed. Since most of the eluate 90 is an organic solvent represented by alcohol, the eluate 90 can be sufficiently volatilized in the above temperature zone.

Note that Figs. 1 to 3 illustrate an example in which the cartridge 20 of a syringe type is filled with the solid phase 10, but the shape of the cartridge 20 is not limited to this, and may be any shape such as a spinning-top type or a disk type. In addition, instead of the cartridge 20, a predetermined column may be filled with the solid phase 10.

In addition, in the method of the present embodiment, it does not necessarily need to perform re-concentration of the eluate 90 by blowing nitrogen (that is, the step (3) described above). In this case, a working time can be shortened although the concentration rate decreases as compared with a case where nitrogen is blown. Note that when the nitrogen blowing is omitted, in order to compensate for the decrease in the concentration rate, the amount of the organic solvent in the step (2) is desirably set to be smaller (for example, an amount of about 1/400 to 1/100 of the sewage sample 30) than that in a case of performing the nitrogen blowing.

A virus concentrate (that is, the eluate 90 recovered in the above (2) or the eluate re-concentrated by the nitrogen blowing in the above (3)) obtained by the method of the present embodiment can be subjected to virus detection by the nucleic acid amplification method. For example, when a virus to be detected is a DNA virus, a DNA extraction processing is performed to the virus concentrate, and then amplification and detection of the DNA is performed by PCR using a primer specific to the virus to be detected. In addition, when the virus to be detected is an RNA virus, the RNA extraction processing is performed on the virus concentrate, and then, by RT-PCR using a primer specific to the virus to be detected, synthesis of a complementary DNA (cDNA) complementary to the RNA is performed and the amplification and the detection of the DNA is performed. Note that an existing reagent kit can be used for the DNA extraction processing, the RNA extraction processing, PCR, and RT-PCR. In addition, detection of an amplification product by PCR or RT-PCR may be performed by a conventional method of electrophoresing the amplification product to confirm the presence or absence and density of a band, but it is desirable to perform the detection by real-time PCR which has been widely used in recent years. The real-time PCR is a method for measuring a DNA concentration by detecting a fluorescence signal generated by interaction between an amplified DNA fragment and a reagent in real time, and can quickly quantify an amplification product by PCR or RT-PCR. In the method of the present embodiment, since at least some of the contaminants 50 contained in the sewage sample 30 can be removed, the virus detection accuracy can be improved by causing the virus concentrate obtained by the method of the present embodiment to be subjected to the virus detection by PCR or RT-PCR.

As described above, since the method of the present embodiment uses the solid phase extraction method, as compared with the method for concentrating a virus sample using the above-described conventional PEG precipitation method, the operation is simple, the time required for virus concentration can be significantly shortened, and a high concentration rate can be achieved. Specifically, in the virus concentration by the conventional PEG precipitation method, it takes about 12 hours to 24 hours to complete the concentration, but in the method of the present embodiment, the concentration of a virus sample can be completed in about 2 hours. In addition, in the method of the present embodiment, the concentration rate is about 2.5 times to 5 times higher than that in the PEG precipitation method. Furthermore, in the method of the present embodiment, an organic solvent is used as an elution solvent, and the organic solvent can be easily evaporated by nitrogen blowing, so that the eluate can be easily re-concentrated. Therefore, a higher concentration rate can be achieved in a shorter time than in a case of a concentration method of a virus sample by the above-described conventional solid phase extraction method (see, for example, Patent Literature 1).

### EXAMPLE

Concentration of a virus sample and detection of RNA derived from the virus were performed by the following procedures. Note that the number of tests was n = 3. First, 100 µL (9.3 × 10⁴ copies) of SARS-CoV-2 Stock (Zepto Metrix), which was an inactivated novel coronavirus, was added to 100 mL of a sewage sample, and a supernatant was collected by performing centrifugal separation at 3,000 rpm for 5 minutes. Then, concentration of the virus sample was performed to the supernatant by the method according to the present invention. The concentration of the virus sample was performed as follows using the HLB solid phase. That is, first, conditioning of the solid phase was performed by passing 10 mL of methanol and 10 mL of ultrapure water through the solid phase in this order, and then the supernatant was passed through the solid phase at a flow rate of 5 to 10 mL/min. Thereafter, the solid phase was washed with 10 mL of ultrapure water, and then the virus was eluted from the solid phase using 2 mL of methanol. From the obtained eluate, 50 µL was fractionated, and 5 µL of RNase-free water was added to the fractionated eluate, and then, the fractionated eluate was blown with nitrogen while being heated at 40 °C to be re-concentrated to 5 µL. Subsequently, an Ampdirect 2019 nCoV detection kit (Shimadzu Corporation), which is a detection reagent for a novel coronavirus, was added to the re-concentrated sample, and RT-PCR was performed on the sample. For RT-PCR, Light Cycler 96 (Roche) was used, and fluorescence derived from the amplification product was measured in real time.

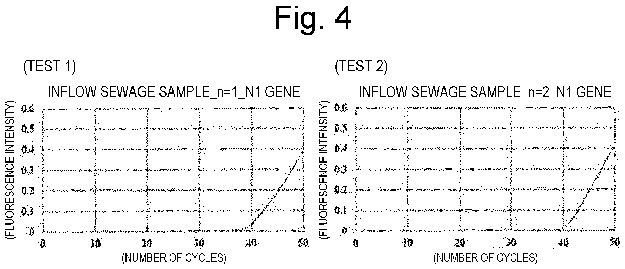

As a result of the above, as illustrated in Fig. 4, amplification of the N1 gene specific to the novel coronavirus was observed in two tests among the three tests. From this, it was found that the eluate obtained by the solid phase extraction in the present example contained the novel coronavirus or its RNA. According to the present example, it has been experimentally confirmed that an enveloped virus represented by a novel coronavirus can be adsorbed on a polymer solid phase having a hydrophobic group such as an HLB solid phase, but it is estimated that a virus other than the enveloped virus can also be adsorbed on a similar solid phase. Note that the HLB solid phase used in the present example is a solid phase having both a hydrophobic group and a hydrophilic group, but it is considered that a solid phase having only a hydrophobic group can also be used as a solid phase in the method for concentrating a virus according to the present invention in the same manner as described above.

### [Various Modes]

It will be understood by those skilled in the art that the exemplary embodiments described above are specific examples of the following modes.

(Clause 1) A method according to a mode of the present invention is a method for concentrating a virus sample, including
bringing a liquid containing a virus into contact with a solid phase made of a polymer having a hydrophobic group to allow the solid phase to retain the virus, and then bringing an organic solvent into contact with the solid phase retaining the virus to cause the virus to be eluted from the solid phase.

(Clause 2) In the method for concentrating a virus sample according to Clause 1, the organic solvent may be an alcohol solvent.

(Clause 3) The method for concentrating a virus sample according to Clause 1 or 2 may further include
evaporating at least part of the organic solvent contained in an eluate eluted from the solid phase by blowing nitrogen onto the eluate.

(Clause 4) In the method for concentrating a virus sample according to any one of Clauses 1 to 3,
the virus may be an enveloped virus.

Since the method for concentrating a virus sample according to Clause 1 uses the solid phase extraction method, as compared with the method for concentrating a virus sample using the above-described conventional PEG precipitation method, the operation is simple, the time required for virus concentration can be significantly shortened, and a high concentration rate can be achieved. In addition, in the method for concentrating a virus sample according to Clause 1, by using an organic solvent for elution of a virus from a solid phase, it is expected that at least some of the viruses are detoxified by the organic solvent, and an effect that a risk of viral infection of a practitioner can be reduced is obtained.

With the method for concentrating a virus sample according to Clause 3, it is possible to further increase the virus concentration in the eluate in a short time by a simple operation.

### REFERENCE SIGNS LIST

- 10: Solid Phase
- 20: Cartridge
- 30: Sewage Sample
- 40: Virus
- 50: Contaminants
- 70: Organic Solvent
- 80: Recovery Container
- 90: Eluate

## Claims

1. A method for concentrating a virus sample, comprising bringing a liquid containing a virus into contact with a solid phase made of a polymer having a hydrophobic group to allow the solid phase to retain the virus, and then bringing an organic solvent into contact with the solid phase retaining the virus to cause the virus to be eluted from the solid phase.

2. The method for concentrating a virus sample according to claim 1, wherein the organic solvent is an alcohol solvent.

3. The method for concentrating a virus sample according to claim 1, further comprising evaporating at least part of the organic solvent contained in an eluate eluted from the solid phase by blowing nitrogen onto the eluate.

4. The method for concentrating a virus sample according to claim 2, further comprising evaporating at least part of the organic solvent contained in an eluate eluted from the solid phase by blowing nitrogen onto the eluate.

5. The method for concentrating a virus sample according to claim 1, wherein the virus is an enveloped virus.

6. The method for concentrating a virus sample according to claim 2, wherein the virus is an enveloped virus.

7. The method for concentrating a virus sample according to claim 3, wherein the virus is an enveloped virus.

8. The method for concentrating a virus sample according to claim 4, wherein the virus is an enveloped virus.
